# EUROPÄISCHE PATENTANMELDUNG

(11) **EP 2 316 917 A1**
(43) Veröffentlichungstag der Anmeldung: **04.05.2011**
(21) Anmeldenummer: 09174928.3
(22) Anmeldetag: 03.11.2009
(51) Int. Cl.: C12N 1/20, C12M 1/00, C12N 1/12

(54) **Verfahren zur mixotrophen Kultivierung von Mikroorganismen und/oder Zellen**

(71) Anmelder: HF Biotec Berlin GmbH, 10117 Berlin (DE)
(72) Erfinder: Hillbrecht, Berit, 15366 Hoppegarten (DE); Follmann, Heinrich, 10117 Berlin (DE)
(74) Vertreter: Rasch, Dorit

(57) **Zusammenfassung**

Die Erfindung betrifft ein Verfahren zur mixotrophen Kultivierung von Mikroorganismen und/oder Zellen, wobei die Mikroorganismen und/oder Zellen auf einem Substrat immobilisiert werden, das mit anorganischem Carbonat beladen ist und anschließend aus dem anorganischen Carbonat durch Zugabe einer schwachen organischen Säure während der Kultivierung CO₂ freigesetzt wird, welches der autotrophen Ernährung unter Lichteinstrahlung dient. Die heterotrophe Ernährung der Mikroorganismen und/oder Zellen erfolgt durch das Anion der schwachen organischen Säure. Die Erfindung betrifft weiterhin eine Vorrichtung zur Durchführung des Verfahrens.

## Beschreibung

Die Erfindung betrifft ein Verfahren zur mixotrophen Kultivierung von Mikroorganismen und/oder Zellen, wobei die Mikroorganismen und/oder Zellen auf einem Substrat immobilisiert werden, das mit anorganischem Carbonat beladen ist und anschließend aus dem anorganischen Carbonat durch Zugabe einer schwachen organischen Säure während der Kultivierung CO₂ freigesetzt wird, welches der autotrophen Ernährung unter Lichteinstrahlung dient. Die heterotrophe Ernährung der Mikroorganismen und/oder Zellen erfolgt durch das Anion der schwachen organischen Säure. Die Erfindung betrifft weiterhin eine Vorrichtung zur Durchführung des Verfahrens.

Mit dem begrenzten Vorrat an fossilen Energieträgern wie Öl und Gas und der mit der Kraftstoffverbrennung in Zusammenhang gebrachten globalen Erwärmung gewinnen regenerative Verfahren zur Erzeugung von Energie zunehmend an Bedeutung. Auf Grund der hohen Wachstumsraten im Vergleich zu herkömmlichen Pflanzen stellt die Kultivierung von Mikroalgen einen viel versprechenden Ansatz dar. Die mögliche Energieausbeute ist bei den Mikroalgen beispielsweise 8 bis 25 mal größer als etwa bei Palmöl und etwa um den Faktor 40 bis 120 größer als bei Rapsöl. Eine direkte Anwendung von Algenbiomasse gilt der Energieerzeugung durch die Nutzung der Algenbiomasse als Substrat für die Herstellung von Biogas durch Fermentation.

Im Stand der Technik sind verschiedene Technologien zur Massenkultivierung phototropher Mikroorganismen und/oder Zellen bekannt. Beschrieben wurden beispielsweise offene Becken oder raceway ponds, die sich unter freiem Himmel befinden und in welchen der CO₂ Eintrag als Lufteintrag mittels paddle-wheels erfolgt. Das Sonnenlicht bietet dabei das für das Wachstum der Mikroorganismen und/oder Zellen erforderliche Licht. Beispielsweise offenbart die DE 10 2004 028 356 A1 ein Verfahren zur ganzjährigen Erzeugung von Sauerstoff bei einem kontinuierlichen Verbrauch von CO₂. Dabei werden Mikroalgen in offenen Becken in einer Nährlösung bestehend aus Wasser, Kalisalzen und Gülle kultiviert. Als Kohlenstoffquelle wird Natriumhydrogencarbonat oder CO₂ aus Abgasen genutzt. Zur Lichtversorgung der Algen wird das Sonnenlicht durch Kunstlicht, welches mittels alternativer Stromquellen erzeugt wurde, ergänzt. Die Nachteile solcher Anlagen sind jedoch beachtlich. Eine Reinkultur ist praktisch nicht möglich, da die ständige Gefahr der Kontamination besteht. Darüber hinaus sind eine gleichmäßige Belichtung der Mikroorganismen und/oder Zellen durch deren gegenseitige Beschattung und wechselhafte Belichtungswinkel sowie eine Temperaturkontrolle in diesen Systemen nicht optimal oder gar nicht möglich.

Um Algen effizienter zu kultivieren, wurden geschlossene Photobioreaktoren entwickelt, die darauf abzielen, die Produktionsraten zu erhöhen und die benötigten Flächen zu verringern. Geschlossene Photobioreaktoren bieten mehrfach Vorteile. Beispielsweise sinken die CO₂-Verluste und das Kontaminationsrisiko. Gleichzeitig werden die Bedingungen für die Kultivierung kontrollier- und steuerbar und die Anlagen verbrauchen weniger Fläche.

Grundsätzlich lassen sich derartige Photobioreaktoren in zylindrische Behälter, Röhrenreaktoren und Plattenreaktoren untergliedern. Als Kohlenstoffquelle wird häufig CO₂-Gas in die Kulturen eingeleitet. Zur Verteilung des Gases in den flüssigen Medien sind behälterförmige Bioreaktoren in der Regel mit Rührern oder ähnlichem zur Bewegung des Kulturmediums ausgestattet. Das bedeutet jedoch einen erheblichen zusätzlichen technischen Aufwand sowie eine Scherbelastung für die meist empfindlichen Zellen der phototrophen Organismen.

Neben der Gaszufuhr ist eine gleichmäßige und optimale Beleuchtung der phototrophen Organismen nötig. Gerade in größeren und damit tieferen Reaktoren stellt die abnehmende Lichtintensität bei zunehmender Schichtdicke und die Beschattung der unteren Schichten durch die Organismen der oberen Schichten ein großes Problem dar. Um eine bessere Beleuchtung zu erreichen sind daher Behälter entwickelt worden, bei denen das Licht über Lichtleiter eingeführt und verteilt wird.

Um die Beleuchtungsbedingungen zu verbessern kann alternativ die Kultivierung phototropher Organismen und Zellkulturen in Photobioreaktoren durchgeführt werden, die als photosynthetische und hydrodynamische Dünnschichtsysteme ausgebildet sind. Die EP 0 968 273 B1 beschreibt beispielsweise ein solches System, bei dem die Kultivierung der Algen in mindestens einem Modul mit einer Vielzahl von parallel zueinander verlaufenden röhrenförmigen Dünnschichtgefäßen erfolgt.

Die DE 198 14 253 C2 beschreibt ebenfalls ein Verfahren zur Herstellung von Biomasse mittels Photosynthese in röhrenförmigen Dünnschichtgefäßen. Durch Beimpfen einer Nährlösung wird eine Kultursuspension erzeugt, in welche ein CO₂-haltiges Gas, vorzugsweise aus Verbrennungsprozessen eingeleitet wird, wobei Blasenbildung in der Kultur vermieden werden soll. Diese Suspension wird anschließend in einen Reaktor geleitet, in dem bei Belichtung der Algensuspension und unter Einstellung eines optimalen Strömungsprofils eine Abreicherung des CO₂-haltigen Gases erfolgt. Um die Ausbeute an Biomasse zu erhöhen werden die Algen mixotroph kultiviert, d.h. es werden weitere Kohlenstoffquellen, wie Einfach- und Mehrfachzucker, beispielsweise Glucose in die Kultursuspension gegeben.

Kennzeichnend für alle derartigen Systeme ist, dass hohe Strömungsgeschwindigkeiten erforderlich sind, um Photoinhibition infolge der erforderlichen hohen Strahlungsintensität zu vermeiden. Diese Strömungen wirken sich kontraproduktiv auf das Wachstum verschiedener Mikroalgen aus. Zudem wird versucht, die Röhren so eng wie möglich parallel anzuordnen, um die Lichtenergie best möglich auszunutzen. Dies gelingt allerdings nur ungenügend, da Krümmer, deren Innenradius praktisch null ist, technisch nicht angefertigt werden können.

Allen nach Stand der Technik beschriebenen Verfahren ist zudem eigen, dass das CO₂ aus einem "Abfallprodukt", wie Biogas oder als technisches Gas bezogen wird. Die dadurch mit in die Kultivierungssuspension eingebrachten Verunreinigungen hemmen die Stoffwechselaktivität der phototrophen Organismen und müssen im Falle von Präzipitaten nach der Ernte von der Biomasse aufwendig abgetrennt werden. Zudem ist in allen Fällen die Lichtausnutzung nicht optimal.

Es ist somit die Aufgabe der vorliegenden Erfindung die Biomasseproduktion weiter zu steigern und gleichzeitig die zur Verfügung stehende oder einzusetzende Lichtenergie maximal auszunutzen. Dabei soll eine verfahrenstechnisch einfache Lösung geschaffen werden, die sowohl eine einfache und kostengünstige Kultivierungsseinrichtung als auch einfache und robuste Kultivierungsbedingungen umfasst. Es ist eine weitere Aufgabe der Erfindung die Photosynthese während der Kultivierung mittels kostengünstiger Verfahren so zu steuern, dass eine maximale Ausbeute an Wertstoffen, insbesondere an Ölen, erzielt wird.

Eine weitere Aufgabe der Erfindung besteht darin, Biogas als Kohlenstoffquelle für die Erzeugung von Biomasse zu verwenden. Dabei sollte eine Möglichkeit gefunden werden, die es erlaubt, das im Biogas befindliche CO₂ zu nutzen, ohne das Biogas durch den bei der Photosynthese entstehenden Sauerstoff zu kontaminieren.

Erfindungsgemäß werden die Aufgaben mit einem Verfahren nach Anspruch 1 gelöst. Die Erfindung stellt somit ein Verfahren zur mixotrophen Kultivierung von Mikroorganismen und/oder Zellen bereit, das die folgenden Schritte umfasst: Aufbringen einer Schicht einer Hohlfaser-Suspension auf einem Träger, Immobilisieren einer mixotrophen Mikroorganismen- und/oder Zellkultur auf der aufgebrachten Hohlfaserschicht, wobei sich die Mikroorganismen und/oder Zellen in der logarithmischen Wachstumsphase befinden, Zugabe einer schwachen organischen Säure mit einem pKs-Wert ≥ 3,8, deren Anion von den mixotrophen Mikroorganismen und/oder Zellen als Substrat verwertet wird, Belichten der Träger mit Tageslicht und/oder Kunstlicht, optional mehrfaches Wiederholen der Zugabe der schwachen organischen Säure und des Belichtens des Trägers und Abernten der mixotrophen Mikroorganismen und/oder Zellen. Erfindungsgemäß ist das Lumen der Hohlfasern mit mindestens einem anorganischen Carbonat gefüllt und/oder äußerlich beladen. Vorzugsweise liegt das anorganische Carbonat in mikrokristalliner Form vor.

In einer besonders bevorzugten Ausführungsform wird zuerst die schwache organische Säure und dann die Mikroorganismen- und/oder Zellkultur auf die Hohlfasersuspension aufgebracht. Vorteilhafterweise wird dadurch nach dem Auftragen der Mikroorganismen und/oder Zellen eine sofortige Belichtung möglich, so dass die Mikroorganismen und/oder Zellen in der logarithmischen Wachstumsphase gehalten werden.

Erfindungsgemäß werden die Mikroorganismen und/oder Zellen also mixotroph und immobilisiert, d.h. trägerfixiert kultiviert. Dadurch kann vorteilhafterweise eine maximale Vergrößerung des Fläche-Volumenverhältnisses erreicht werden. Als Kohlenstoffquelle kommen bei dem erfindungsgemäßen Verfahren sowohl CO₂ als auch organische Verbindungen zum Einsatz. Das CO₂ wird in Form von anorganischen Carbonaten auf dem Träger gespeichert und in der Kultur durch Zugabe der schwachen organischen Säure zum Verbrauch wieder freigesetzt.

In einer bevorzugten Ausgestaltung der Erfindung werden als Hohlfasern Zellulosefasern, insbesondere Zellstofffasern, Hefehohlfasern, Zeolithe oder andere Substrate mit Hohlräumen verwendet. In einer besonders bevorzugten Ausführungsform werden Zellstofffasern verwendet. Unter Zellstoff wird die beim chemischen Aufschluss von Faserpflanzen, wie z.B. Holz, entstehende Masse, die vorwiegend aus Zellulose besteht, verstanden. Dabei wird zwischen aus Hartholz, wie z.B. Birke, Buche oder Eukalyptus hergestellten Kurzfasern und den aus Weichholz, wie z.B. Kiefer, Fichte oder anderen Nadelhölzern, hergestellten Langfasern unterschieden. Erfindungsgemäß werden wahlweise Langfasernund/oder Kurzfasern verwendet. Die Ausdehnung entlang der Längsachse erfindungsgemäß geeigneter Hohlfasern liegt vorzugsweise im Bereich von 0,5 bis 10 µm, insbesondere im Bereich von 1 bis 5 µm.

Das Lumen, d.h. der Hohlraum der Zellstofffasern ist mit einem anorganischen Carbonat, vorzugsweise in mikrokristalliner Form, gefüllt. Der Füllstand liegt vorzugsweise im Bereich von 10 bis 80%, weiter bevorzugt im Bereich von 20 bis 80%, besonders bevorzugt im Bereich von 30 bis 50%. In einer bevorzugten Ausgestaltung der Erfindung wird das Carbonat im Lumen der Hohlfasern ausgefällt. Vorzugsweise wird das anorganische Carbonat im Lumen der Hohlfasern aus der Reaktion von CO₂ mit Erdalkalihydroxiden, insbesondere mit Ca(OH)₂ und/oder Mg(OH)₂, gebildet. Als CO₂-Quelle wird vorzugsweise CO₂ aus dem Abgasstrom einer Biogasanlage verwendet. Beispielsweise entsteht durch die Reaktion des im Biogas enthaltenen CO₂ mit Kalkmilch oder Magnesiumhydroxiden, precipitated calcium carbonate (PCC), Magnesiumcarbonat oder deren Mischcarbonate. Die Herstellung von PCC unter Nutzung des im Biogas enthaltenen CO₂ ist beispielsweise in der WO 2008/128776 beschrieben. Durch die Fällungsreaktion wird die Gasphase eines Methanreaktors mittels des "PCC-Verfahrens" in eine kristalline CaCO₃-Phase und eine gasförmige quasi nur Methan enthaltene Phase, die direkt zur Energiegewinnung eingesetzt werden kann, aufgetrennt. Die Fällungsreaktion des CO₂ erfolgt nach der allgemeinen Formel:

CO₂ +Me(OH)₂ ⇔ MeCO₃ + H₂O,

wobei Me ein Erdalkalimetall ist.

Alternativ kann das anorganische Carbonat ausgefällt, abgetrennt und dann mit einer Suspension aus Zellulosefasern, insbesondere einer Zellstofffasersuspension, gründlich vermischt werden, wobei neben der äußeren Wandung auch die Faserhohlräume mit dem anorganischen Carbonat beladen werden. Das anorganische Carbonat dient als CO₂-Speicher, welches dann über ein einfaches Säure-Base-Gleichgewicht kontrolliert freigesetzt werden kann.

Erfindungsgemäß wird eine Schicht der mit dem anorganischen Carbonat gefüllten Hohlfasern als Suspension auf einem Träger aufgetragen. Vorzugsweise liegt die Schichtdicke der Hohlfaser-Suspension im Bereich von 0,1 bis 50 mm, vorzugsweise im Bereich von 1 bis 10 mm und besonders bevorzugt im Bereich von 1 bis 5 mm. Als Träger kommen alle dem Fachmann bekannten Materialien in Frage, die in Zellkulturen oder Bioreaktoren eingesetzt werden können und gleichzeitig eine ausreichende mechanische Festigkeit liefern. Beispielsweise besteht der Träger aus Glas, Gummi, Metall, Kunststoff oder textilem Gewebe, vorzugsweise aus Kunststoff. Besonders bevorzugt ist der Träger als ebene oder schiefe Fläche, vorzugsweise als Trägerplatte ausgestaltet. Der Auftrag der einzelnen Substanzen, wie beladene Hohlfaser-Suspension, Mikroorganismen- und/oder Zellkultur und schwache organische Säure erfolgt vorzugsweise ganzflächig. Bei größeren Flächen, kann auch eine Unterteilung in kleinere Einheiten vorgenommen werden.

Nach dem Auftragen der Hohlfaser-Suspension auf dem Träger wird auf der Hohlfaserschicht eine mixotrophe Mikroorganismen- und/oder Zellkultur immobilisiert. Dazu werden vorzugsweise mixotrophe Mikroorganismen und/oder Zellen aus einer üblichen Vorkultur auf die Hohlfasern aufgebracht. Geeignete Vorkulturen sind dem Fachmann bekannt. Üblicherweise werden die Mikroorganismen und/oder Zellen in der Vorkultur unter autotrophen oder mixotrophen Bedingungen kultiviert. Geeignete Kohlenstoffquellen sind beispielsweise Einfach- und/oder Mehrfachzucker, wie z.B. Glucose. Das Grundmedium ist entsprechend dem verwendeten Mikroorganismus bzw. der verwendeten Zelle auszuwählen und enthält die benötigten Makro- und Mikronährstoffe.

Erfindungsgemäß geeignete Vorkulturen weisen vorzugsweise eine Zelldichte im Bereich von 0,01 bis 10g/l, vorzugsweise im Bereich von 0,05 bis 5 g/l, weiter bevorzugt im Bereich von 0,1 bis 1 g/l auf. Erfindungsgemäß sind die Mikroorganismen und/oder Zellen beim Überimpfen in der logarithmischen Wachstumsphase. Dadurch kann ein Einbruch des Wachstums verhindert werden. Zur Kultivierung der Mikroorganismen und/oder Zellen auf den Hohlfasern wird lediglich ein dünner Flüssigkeitsfilm aus Kulturmedium benötigt, der die Kulturen feucht hält und mit weiteren Nährstoffen versorgt. Die Stärke des Flüssigkeitsfilms liegt vorzugsweise im Bereich von 0,1 bis 10 mm, weiter bevorzugt im Bereich von 1 bis 5 mm. Der Flüssigkeitsfilm kann entweder beim Überimpfen aus der Vorkultur stammen oder die Mikroorganismen und/oder Zellen werden in einem geringerem Volumen überimpft und anschließend mit einem Kultivierungsmedium überschichtet. Das Kultivierungsmedium entspricht in diesem Falle vorzugsweise dem Medium der Vorkultur mit Ausnahme der alternativen Kohlenstoffquellen, d.h. den Einfach- oder Mehrfachzuckern. Optimale Kultivierungsbedingungen liegen im Bereich von 20 bis 40 °C, vorzugsweise im Bereich von 25 bis 30 °C.

Zur Ernährung der Mikroorganismen und/oder Zellen wird das CO₂ aus den anorganischen Carbonaten mittels einer schwachen organischen Säure wieder freigesetzt. Dazu wird die schwache organische Säure dem Medium zugegeben oder der Träger mit der schwachen organischen Säure besprüht. Dabei wird die schwache organische Säure derart ausgewählt, dass Ihr Anion gleichzeitig ein Substrat für die Mikroorganismen und/oder Zellen darstellt.

In einer bevorzugten Ausführungsform der Erfindung wird eine schwache organische Säure mit einer Konzentration im Bereich von 0,001 bis 1 mol/l, vorzugsweise im Bereich von 0,01 bis 0,5 mol/l, weiter bevorzugt im Bereich von 0,01 bis 0,3 mol/l verwendet. Die Dosierung der schwachen organischen Säure erfolgt an Hand des Wachstums der Mikroorganismen und/oder der Zellen und wird zudem an die Zelldichte angepasst, d.h. bei größeren Ausgangszelldichten und schnellerem Zellwachstum wird eine höhere Molarität der schwachen organischen Säure gewählt. Dabei werden die Verhältnisse der Konzentration an organischer Säure so auf die Konzentration der zuvor aufgebrachten Mikroorganismen und/oder Zellen abgestimmt, dass die spezifische Wachstumsrate der Mikroorganismen und/oder Zellen, noch nicht von der organischen Säure gehemmt wird, sondern gerade durch eine optimale Freisetzung der Kohlenstoffquellen maximal wird. Vorteilhafterweise wird zusätzlich durch die leichte Ansäuerung des Kulturmediums ein effektiver Säureschutz gegenüber unerwünschten Keimen, wie anderen Mikroorganismen oder Pilzen erzielt und somit das Infektionsrisiko der Kultur beherrschbar.

Die schwache organische Säure diffundiert in das Lumen der Zellstofffasern, reagiert dort mit den anorganischen Carbonaten, beispielsweise CaCO₃ und setzt dabei CO₂ nach der allgemeinen Formel

CaCO₃ + 2 HSäureanion ⇔ CaSäureanion + CO₂ + H₂O frei.

Das CO₂ diffundiert aus dem Lumen in die darüber befindliche Kulturlösung und wird von den Mikroorganismen und/oder Zellen unter Einfluss von Licht autotroph verwertet. Das Säureanion bildet mit dem Erdalkaligegenion die alternative Kohlenstoffquelle, die von den Mikroorganismen und/oder Zellen heterotroph verwertet wird. Insgesamt liegt erfindungsgemäß somit ein mixotropher Stoffwechsel bei den Mikroorganismen und/oder Zellen vor. Gleichzeitig mit der Verstoffwechselung der Substrate erfolgt ein Aufbau an Biomasse.

Erfindungsgemäß wird unter schwacher organischer Säure jede organische Säure mit einem pKs-Wert ≥ 3,8, vorzugsweise mit einem pKs-Wert ≥ 4,5 verstanden. In einer bevorzugten Ausgestaltung der Erfindung ist die schwache organische Säure, Essigsäure, Zitronensäure, Isozitronensäure, Oxalsäure, Milchsäure, Gluconsäure, Adipinsäure, Bernsteinsäure, Valeriansäure, Itaconsäure, cis-Aconitsäure oder eine Mischung davon. In einer besonders bevorzugten Ausgestaltung ist die schwache organische Säure Essigsäure. Die Essigsäure reagiert mit dem Calciumcarbonat zu Calciumacetat.

In einer weiteren bevorzugten Ausführungsform wird die schwache organische Säure mit den zum Zellwachstum benötigten Mineralstoffen und Spurenelementen versetzt. Vorteilhafterweise werden dadurch die schwerlöslichen Elemente von der Säure komplexiert und können in höheren Konzentrationen zugegeben werden, ohne dass eine Präzipitation, wie beispielsweise von Ca₃(PO₄)₂, erfolgt.

Für die autotrophen Stoffwechselprozesse benötigen die Mikroorganismen und/oder Zellen Licht. Daher werden die Mikroorganismen und/oder Zellen erfindungsgemäß mit Tages- und/oder Kunstlicht belichtet. Die Belichtungsverhältnisse werden dabei so gewählt, dass tagsüber das zur Verfügung stehende standortspezifische Licht verwertet wird und nachts mit Kunstlicht belichtet wird, dessen Wellenlänge auf die Erfordernisse der Fotosynthese ausgerichtet ist. Vorzugsweise wird Kunstlicht mit Wellenlängen im Bereich von 670 bis 900 nm, vorzugsweise im Bereich von 670 bis 700 nm verwendet. In einer bevorzugten Ausgestaltung der Erfindung erfolgt die Belichtung abwechselnd mit Tages- und mit Kunstlicht. Ein bevorzugter Wechsel der Belichtung erfolgt nach 12h. Die durchschnittliche zur Belichtung verwendete Lichtintensität liegt im Bereich von 1 bis 40 W/m², vorzugsweise im Bereich von 5 bis 25 W/m². In einer besonders bevorzugten Ausgestaltung der Erfindung ist die Lichtstärke tagsüber und nachts annähernd gleich.

Die Schritte Zugabe der schwachen organischen Säure, d.h. das zur Verfügung stellen von Kohlenstoffquellen und Belichten des Trägers werden optional mehrfach wiederholt. Vorzugsweise werden die Schritte so oft wiederholt bis die Zelldichte im Bereich von 1 bis 150 g Trockenmasse/l, vorzugsweise im Bereich von 1 bis 100 g Trockenmasse/l, weiter bevorzugt im Bereich von 10 bis 100 g Trockenmasse/l liegt. Die Wachstumsraten ergeben sich in Abhängigkeit von der Säurekonzentration, der optischen Dichte der Kultur und der Lichtintensität. Überraschenderweise wurde festgestellt, dass sich die Wachstumsterme aus der autotrophen und der heterotrophen Kultivierung addieren und somit ein deutlich größerer Aufbau an Biomasse erreicht werden kann. In einer bevorzugten Ausführungsform der Erfindung liegt der autotrophe Anteil der Photosyntheserate im Bereich von 1/10 bis 3/5, vorzugsweise im Bereich von 1/5 bis 2/5, der Absorptionsrate der organischen Kohlenstoffquelle.

Ist die gewünschte Zelldichte erreicht, werden die Mikroorganismen und/oder Zellen abgeerntet. Dem Fachmann sind gängige Verfahren zum Ernten von immobilisierten Mikroorganismen und/oder Zellen bekannt. Vorzugsweise werden die Mikroorganismen und/oder Zellen mechanisch geerntet, beispielsweise abgeschabt. Anschließend werden die Mikroorganismen und/oder Zellen einer weiteren Verwertung, vorzugsweise der Gewinnung von Lipiden, zugeführt. Bezogen auf die eingestrahlte Lichtintensität kann durch das erfindungsgemäße Verfahren eine 10-fache Steigerung des Wirkungsgrads im Vergleich zu herkömmlichen Photosynthese-basierten Prozessen erreicht werden.

Vorzugsweise handelt es sich bei den Mikroorganismen und/oder Zellen um phototrophe Organismen. Erfindungsgemäß geeignete Mikroorganismen sind Cyanobacterien und erfindungsgemäß geeignete Zellen sind Mikroalgen, die vorzugsweise zur Klasse der *Cryptophyceae, Chlorophyceae, Chrysophyceae, Trebouxiophyceae, Eustigmatophyceae, Bacillariophyceae,* oder *Prymnesiophyceae* gehören. In einer bevorzugten Ausgestaltung werden die Mikroorganismen und/oder Zellen als Monokulturen oder als Mischkulturen verwendet. Zu den besonders bevorzugten Mikroorganismen und/oder Zellen gehören *Cryptomonas sp., Chlamydomonas sp., Scenedesmus sp., Haematococcus sp., Botryococcus sp., Tetraselmis sp., Dunaliella sp., Neochloris sp., Dinobryon sp., Chlorella sp., Nannochloropsis sp., Nitzschia sp., Chaetoceros sp., Phaeodactylum sp., Pleurochrysis sp., Isochrysis sp., Arthrospira sp.* oder Mischungen davon. Besonders bevorzugt wird erfindungsgemäß *Chlorella sp.* verwendet.

In einer besonders bevorzugten Ausgestaltung der Erfindung wird die Essigsäure in einem parallelen Fermentationsprozess hergestellt. Dabei wird die Essigsäure vorzugsweise durch Fermentation organischer Abfallstoffe, insbesondere durch Fermentation mittels eines Bakteriums der Familie der *Clostridiaceae* oder *Gluconobacter* und besonders bevorzugt durch Fermentation mittels *Clostridium acetobutylicum* oder *Gluconobacter oxydans* hergestellt. Als organische Abfallstoffe können erfindungsgemäß beliebige organische Abfallstoffe verwendet werden. Vorzugsweise werden industrielle Abfallstoffe wie z.B. Vinasse, Glycerin oder Schlempe verwendet. Die bei der Fermentation als Beiprodukt entstehende Buttersäure muss von der Essigsäure abgetrennt werden. Vorzugsweise erfolgt die Abtrennung der Essigsäure mittels Membranfiltration. Weitere Reaktionsprodukte, wie ein Gemisch aus CO₂ und H₂, können nach der Überführung in die Gasphase einer geeigneten Brennstoffzelle zugeführt werden. In einer bevorzugten Ausgestaltung der Erfindung liefert diese Brennstoffzelle die Energie, die zur Belichtung mit Kunstlicht während der Kultivierung benötigt wird. Die Reststoffe aus der Clostridien- oder Gluconobacterfermentation können kontinuierlich einem Methanreaktor zugeführt und in die üblichen Produkte, wie Biogas und Gärrest umgewandelt werden.

In einer weiteren bevorzugten Ausgestaltung der Erfindung wird das Verfahren kontinuierlich durchgeführt. Vorzugsweise wird dabei ein Träger zur Kultivierung der Mikroorganismen und/oder Zellen verwendet, der beweglich ist. Vorzugsweise wird ein Transportband oder Laufband als Träger verwendet. Dabei werden am Beginn des Transportbandes kontinuierlich die mit dem anorganischem Carbonat beladene Hohlfaser-Suspension, und dann nacheinander die schwache anorganische Säure, die Mikroorganismen und/oder Zellen und das Kulturmedium aufgetragen. Anschließend bewegt sich das Transportband kontinuierlich unter den Lichtquellen hindurch, wobei vorzugsweise zuerst die natürliche und anschließend die künstliche Lichtquelle passiert wird. Während der Belichtung sind erfindungsgemäß weitere Zugaben an schwacher anorganischer Säure vorgesehen. Die Geschwindigkeit des Transportbandes ist erfindungsgemäß frei wählbar und wird an die Wachstumsraten der Mikroorganismen und/oder Zellen angepasst. Geeignete Geschwindigkeiten des Transportbandes liegen im Bereich von 0,1 bis 50 cm/h und sind vorzugsweise abgestimmt auf die spezifische Wachstumsrate der Mikroorganismen und/oder Zellen und die bei der Ernte gewünschte Konzentration.

Erfindungsgemäß reagiert das anorganische Carbonat aus den Lumen der Hohlfasern im Laufe der Kultivierung der Mikroorganismen und/oder Zellen vollständig mit der schwachen organischen Säure. Da die dabei entstehenden Reaktionsprodukte aus dem Lumen in die über der Faserschicht befindliche Nährlösung diffundieren, sind die Lumen der Hohlfasern zum Zeitpunkt des Erntens der Mikroorganismen und/oder Zellen wieder frei von mikrokristallinen Carbonaten. Die bei der Verstoffwechselung entstehenden Beiprodukte sollen an Hand der Reaktion von Calciumcarbonat und Essigsäure beispielhaft erläutert werden. Die Mikroorganismen und/oder Zellen verwerten das entstehende Calziumacetat als Kohlenstoffquelle und akkumulieren dabei die Calciumionen im Medium. Diese bilden mit den Anionen aus dem Kulturmedium diverse Salze, wie z.B. Calciumsulfat, Calciumphosphat, Calciumcarbonat oder Calciumhydroxid, die außerhalb der Fasern auskristallisieren und durch verschiedene Trennverfahren, wie z.B. Flotation der Hohlfasern, gut abtrennbar sind. Sowohl die Hohlfasern als auch die Salzpräzipitate können wahlweise in den Prozess zurückgeführt oder als düngemittelwirksame Fraktion aus dem Prozess ausgeschleust werden. Die Auswahl der dem Medium zugeführten Nährsalze wird so vorgenommen, dass sich im Medium praktisch kein Calciumphophat bilden kann und das mengenmäßig bestimmende Anion das Hydroxylion ist. Dadurch wird ab Überschreiten der Löslichkeitsgrenze vorzugsweise das unlösliche Calciumhydroxyd gebildet, das dann abgetrennt und wieder in den Prozess als Ausgangsmaterial zurückgeführt werden kann. Somit ist es erfindungsgemäß vorteilhafterweise möglich, das Verfahren als Kreisprozess auszulegen. Nach jedem Abernten der Mikroorganismen und/oder Zellen können die Hohlfasern und/oder das ausgefallene Calciumhydroxid abgetrennt werden und die Hohlfasern erneut mit dem Claciumhydroxid beladen und regenerativ in den Prozess als Ausgangsmaterial zurückgeführt werden.

Die Erfindung betrifft weiterhin eine Anlage zur Durchführung des erfindungsgemäßen Verfahrens. Eine erfindungsgemäße Anlage mit einem ersten und einem zweiten Ende weist einen zellkulturgeeigneten Träger auf, der mit einem Antriebsmittel verbunden ist und der Antrieb eine Bewegung des Trägers von dem ersten Ende zum zweiten Ende der Anlage bewirkt. Weiterhin weist die erfindungsgemäße Anlage ein Mittel zum Auftragen einer mit anorganischem Carbonat gefüllten Hohlfasersuspension auf, wobei das Mittel zum Auftragen der Hohlfasersuspension am ersten Ende der Anlage positioniert ist. Ein geeignetes Mittel zum Auftragen der Hohlfasersuspension ist beispielsweise eine Düse, ein Spachtel, eine Bürste oder ein Schaber. Des Weiteren weist die erfindungsgemäße Anlage ein Mittel zum Auftragen einer Mikroorganismen- und/oder Zellkultur auf, wobei das Mittel zum Auftragen der Mikroorganismen- und/oder Zellkultur zwischen dem Mittel zum Auftragen der Hohlfasersuspension und dem zweiten Ende der Anlage positioniert ist. Zudem weist die erfindungsgemäße Anlage ein Mittel zum Auftragen einer schwachen organischen Säure auf, wobei das Mittel zum Auftragen der Säure zwischen dem Mittel zum Auftragen der Hohlfasersuspension und dem Mittel zum Auftragen der Mikroorganismen- und/oder Zellkultur positioniert ist. Ein geeignetes Mittel zum Auftragen der Mikroorganismen- und/oder Zellkultur und/oder der schwachen organischen Säure ist beispielsweise eine Düse und/oder andere Verteiler- und/oder Sprühvorrichtung. Des Weiteren weist die erfindungsgemäße Anlage eine erste Lichtquelle auf, die zwischen dem Mittel zum Auftragen der Mikroorganismen- und/oder Zellkultur und dem zweiten Ende der Anlage positioniert ist. Weiterhin weist die erfindungsgemäße Anlage optional ein weiteres Mittel zum Auftragen einer schwachen organischen Säure auf, das zwischen der ersten Lichtquelle und dem zweiten Ende der Anlage positioniert ist. Zudem weist die erfindungsgemäße Anlage eine zweite Lichtquelle auf, die zwischen der ersten Lichtquelle oder dem optionalen weiteren Mittel zum Auftragen der schwachen organischen Säure und dem zweiten Ende der Anlage positioniert ist. Ebenfalls weist die erfindungsgemäße Anlage ein Mittel zum Ernten der Mikroorganismen- und/oder Zellkultur auf, das am zweiten Ende der Anlage positioniert ist. Zudem weist die erfindungsgemäße Anlage ein Mittel zum Auffangen der Mikroorganismen und/oder Zellen, das benachbart zum Mittel zum Ernten positioniert ist und ein Mittel zum Auffangen der Hohlfasersuspension auf, das am zweiten Ende der Anlage positioniert ist.

Vorzugsweise handelt es sich bei der ersten Lichtquelle um eine natürliche Lichtquelle, d.h. eine Lichtquelle mit einem Tageslichtspektrum und bei der zweiten Lichtquelle um eine künstliche Lichtquelle, d.h. um eine Lichtquelle mit einem vom Tageslicht verschiedenen Lichtspektrum, vorzugsweise einem Lichtspektrum im Bereich von 670 bis 900 nm, besonders bevorzugt im Bereich von 670 bis 700 nm. Erste und zweite Lichtquelle sind vorzugsweise derart positioniert, dass ihr Ausleuchtungsbereich die Mikroorganismen und/oder Zellen vollständig erfasst, sobald sie auf dem mit der Hohlfasersuspension beschichteten, zellkulturgeeigneten Träger aufgetragen wurden. Die Ausleuchtungsbereiche überschneiden sich vorzugsweise nicht.

In einer weiteren bevorzugten Ausgestaltung der Erfindung können optional auch mehrere Mittel zum Auftragen einer schwachen organischen Säure und mehrere erste und zweite Lichtquellen hintereinander angeordnet werden. Die Anzahl der Mittel zum Auftragen der Säure und die Anzahl der Lichtquellen ergibt sich aus der zu erhaltenden Biomasse.

In einer bevorzugten Ausführungsform weist die erfindungsgemäße Anlage einen Fällungsreaktor zur Herstellung der mit anorganischem Carbonat gefüllten

Hohlfasersuspension auf, der mit dem Mittel zum Auftragen der Hohlfasersuspension verbunden ist. In dem Fällungsreaktor wird aus einer wässrigen Me(OH)₂-Lösung MeCO₃ durch Einleiten von CO₂ ausgefällt, wobei Me ein Erdalkalimetall ist. Der Fällungsreaktor weist daher vorzugsweise ein Gaseinleitungsrohr auf. In einer weiteren Ausgestaltung weist der Fällungsreaktor einen Rührer auf, der die Hohlfasersuspension und das ausgefällte MeCO₃ zur Beladung der Hohlfasern durchmischt.

In einer weiteren Ausgestaltung der Erfindung weist die erfindungsgemäße Anlage einen Bioreaktor zur Herstellung der Mikroorganismen- und/oder Zellkultur auf, der mit dem Mittel zum Auftragen der Mikroorganismen- und/oder Zellkultur verbunden ist. Geeignete Bioreaktoren sind dem Fachmann bekannt.

In einer weiteren Ausgestaltung der Erfindung weist die erfindungsgemäße Anlage einen Fermenter zur Herstellung der schwachen organischen Säure auf, wobei der Fermenter mit jedem der in der Anlage vorhandenen Mittel zum Auftragen der schwachen organischen Säure verbunden ist. Vorzugsweise weist der Fermenter zusätzlich eine Membranfiltrationsanlage zur Aufreinigung der schwachen organischen Säure auf.

In einer besonders bevorzugten Ausführungsform der Erfindung weist die Anlage einen Fällungsreaktor, einen Bioreaktor und einen Fermenter auf.
- Fig. 1: zeigt eine Anlage (10) zur Durchführung des erfindungsgemäßen Verfahrens schematisch
- Fig. 2: zeigt eine bevorzugte Ausführungsform der Anlage (10) mit zusätzlichem Fällungsreaktor (40), Bioreaktor (42) und Fermentor (44) schematisch

Im Folgenden soll die Erfindung an Hand von einigen Beispielen näher erläutert werden.

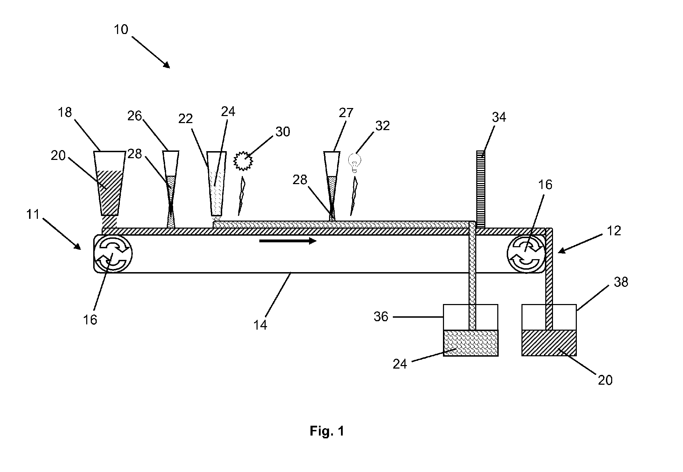

Fig. 1 zeigt eine erfindungsgemäße Anlage 10 zur Durchführung des erfindungsgemäßen Verfahrens schematisch. Die Anlage 10 weist ein erstes Ende 11 und ein zweites Ende 12 auf. Ein zellkulturgeeigneter Träger 14 ist mit einem Antriebsmittel 16 verbunden, das eine Bewegung des Trägers 14 vom ersten Ende 11 zum zweiten Ende 12 der Anlage 10 bewirkt. Vorzugsweise ist der Träger 14 als Transportband aus Kunststoff ausgestaltet. Am ersten Ende 11 der Anlage ist ein Mittel 18 zum Auftragen einer mit anorganischem Carbonat gefüllten Hohlfasersuspension 20 angeordnet. Ein geeignetes Mittel 18 zum Auftragen ist eine Düse, ein Spachtel, eine Bürste oder ein Schaber. Zwischen dem Mittel 18 und dem zweiten Ende 12 der Anlage 10 ist dann ein Mittel 26 zum Auftragen einer schwachen organischen Säure 28 sowie einer Spurenelementelösung, vorzugsweise eine Sprühvorrichtung, angeordnet. Zwischen dem Mittel 26 zum Auftragen der schwachen organischen Säure 28 und dem zweiten Ende 12 der Anlage 10 ist weiterhin ein Mittel 22 zum Auftragen einer Mikroorganismen- und/oder Zellkultur 24 angeordnet. Ein geeignetes Mittel 22 ist beispielsweise eine Düse. Benachbart zu der Düse 22 befindet sich dann eine erste, vorzugsweise natürliche Lichtquelle 30, wobei als natürliche Lichtquelle 30 sowohl eine lichtdurchlässige Verbindung zum Tageslicht, wie Folien oder Glas, als auch eine Glühlampe oder Leuchtdioden (LED) mit Tageslichtspektrum verwendet werden kann. Der Ausleuchtungsbereich erfasst die frisch aufgetragenen Mikroorganismen und/oder Zellen vollständig. Am Ende des Ausleuchtungsbereichs der natürlichen Lichtquelle 30 befindet sich eine weitere Sprühvorrichtung 27 zum Aufsprühen der schwachen organischen Säure. Dann folgt in Richtung des zweiten Endes 12 der Anlage 10 eine zweite, vorzugsweise künstliche Lichtquelle 32 mit einem Wellenlängenbereich von 670 bis 700 nm. Natürliche Lichtquelle 30 und künstliche Lichtquelle 32 sind derart angeordnet, dass sich die ausgeleuchteten Bereiche des Trägers 14 nicht überlappen. Am zweiten Ende 12 der Anlage 10 befindet sich ein Mittel 34 zum Ernten der Mikroorganismen- und/oder Zellkultur 24, vorzugsweise ein mechanischer Schaber, der die Mikroorganismen und/oder Zellen einfach von der Hohlfasersuspension 20 herunter schiebt. Die abgeernteten Mikroorganismen und/oder Zellen werden in einem Mittel 36 zum Auffangen der Mikroorganismen und/oder Zellen gesammelt und dann der Weiterverarbeitung zugeführt. Am zweiten Ende 12 der Anlage 10 verlässt die Hohlfaserschicht 20 automatisch den bewegten Träger 14 und wird in einem Mittel 38 zum Auffangen der Hohlfasersuspension 20 gesammelt. Die Hohlfasersuspension 20 wird ebenfalls der Wiederverwertung zugeführt, erneut mit ebenfalls im Prozess anfallendem Calciumhydroxid beladen und zurück in das Mittel 18 zum Auftragen der mit anorganischem Carbonat gefüllten Hohlfasersuspension 20 gegeben.

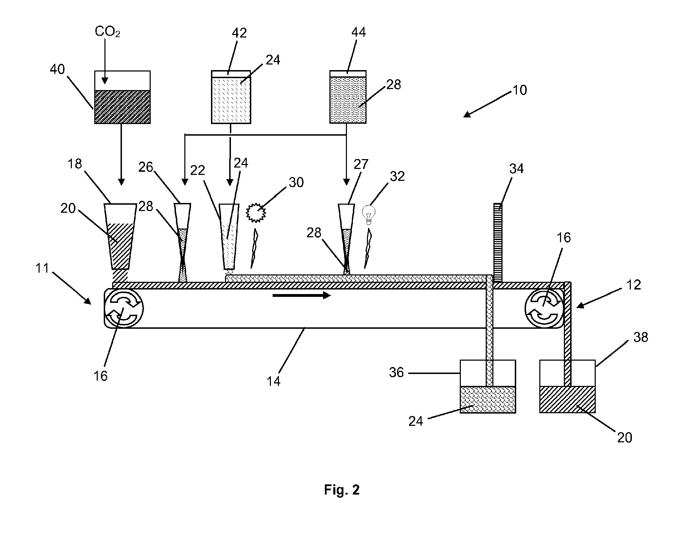

Fig. 2 zeigt eine bevorzugte Ausführungsform der erfindungsgemäßen Anlage 10. Bezugszeichen gelten analog wie in Fig. 1. Zusätzlich zu den in Fig. 1 genannten Bauteilen weist die Anlage 10 einen Fällungsreaktor 40 zur Herstellung der mit anorganischem Carbonat gefüllten Hohlfasersuspension 20 auf, der mit dem Mittel 18 zum Auftragen der Hohlfasersuspension 20 verbunden ist. In dem Fällungsreaktor 40 wird aus einer wässrigen Me(OH)₂-Lösung MeCO₃ durch Einleiten von CO₂ ausgefällt, wobei Me ein Erdalkalimetall ist. Der Fällungsreaktor 40 weist daher vorzugsweise ein Gaseinleitungsrohr oder eine selbstansaugende Turbine auf, die CO₂ intensiv in die Suspension eintragen kann. Als CO₂-haltiges Gas wird vorzugsweise Gas aus einem Bioreaktor verwendet. In einer weiteren Ausgestaltung weist der Fällungsreaktor 40 einen Rührer auf, der die Hohlfasersuspension und das ausgefällte MeCO₃ zur Beladung der Hohlfasern durchmischt.

Weiterhin weist die Anlage 10 einen Bioreaktor 42 zur Herstellung der Mikroorganismen- und/oder Zellkultur 24 auf, der mit dem Mittel 22 zum Auftragen der Mikroorganismen- und/oder Zellkultur 24 verbunden ist. Geeignete Bioreaktoren 42 sind dem Fachmann bekannt.

Zudem weist die Anlage 10 einen Fermenter 44 zur Herstellung der schwachen organischen Säure 28 auf, wobei der Fermenter 44 mit den Mitteln 26, 27 zum Auftragen der schwachen organischen Säure 28 verbunden ist. Vorzugsweise weist der Fermenter 44 zusätzlich eine Membranfiltrationsanlage zur Aufreinigung der schwachen organischen Säure auf 28.

### Beispiel 1

Das erfindungsgemäße Verfahren wurde in einer Anlage 10 nach Fig. 2 durchgeführt. Biogas aus einem Methanreaktor wurde in eine Löschkalksuspension im Fällungsreaktor 40 eingeleitet. Durch die Reaktion von CO₂ und Ca(OH)₂ wurde CaCO₃ gebildet. Anschließend erfolgte die Beladung von Zellstoffhohlfasern mit dem CaCO₃ durch Carboxylieren von Ca(OH)₂ in Gegenwart einer Fasersuspension unter Rühren, vorzugsweise mit einer Frings-Turbine. Die Beladungsrate der Zellstoffhohlfasern betrug im Mittel 20 bis 45 %. Anschließend wurde die beladene Zellstofffasersuspension 20 auf ein Kunststofftransportband 14 bis zu einer Schichtdicke von 3 mm aufgetragen. Der Vorschub des Transportbandes 14 betrug 5 cm/h. Parallel dazu wurden ein Bioreaktor 42 und ein Fermenter 44 betrieben. In dem Bioreaktor 42 wurden *Chlorella sp.* in einem Glucose-haltigen Medium als Vorkultur bei 30 °C kultiviert. Dabei ist zu beachten, dass die Zellen kontinuierlich in der logarithmischen Wachstumsphase gehalten werden müssen. In dem Fermenter 44 wurde mittels *Clostridium acetobutylicum* aus organischen Abfällen Essigsäure 28 produziert und mittels Membranfiltration abgetrennt.

Die Schicht aus beladenen Hohlfasern 20 wurde mit Essigsäure 28 einer Konzentration von 0,01 mol/l besprüht. Anschließend wurde eine Suspension von *Chlorella sp.* 24 aus dem Bioeaktor 42 mit einer Konzentration von 0,3 g Trockenmasse/l aufgesprüht und mit einem dünnen Film (5 mm) aus Kultivierungsmedium bedeckt. Das Kultivierungsmedium entsprach dem Medium der Vorkultur mit Ausnahme der Glucose..

Die Belichtung erfolgte während der ersten 12h der Kultur mit Tageslicht 30 mit einer Beleuchtungsstärke zwischen 5 und 20 W/m² und während der zweiten 12h der Kultur mit Kunstlicht 32 mit Wellenlängen im Bereich von 670 bis 700 nm mit einer Beleuchtungsstärke von 20 W/m².

Die Endkonzentration der Biomasse nach 24h Kultivierung betrug 1 g Trockenmasse/l. Der Metabolisierungsgrad der Essigsäure lag bei mehr als 95% und die Verwertung des freigesetzten CO₂ lag bei mehr als 50%.

### Beispiel 2

Das Verfahren wurde wie in Beispiel 1 durchgeführt. Die Konzentration der Suspension von *Chlorella sp.* aus dem ersten Fermenter betrug 1 g Trockenmasse/l die Konzentration der Essigsäure betrug 0,03mol/l. Die Belichtung erfolgte während der ersten 12h der Kultur mit Tageslicht mit einer Beleuchtungsstärke zwischen 5 und 25 W/m² und während der zweiten 12h der Kultur mit Kunstlicht mit Wellenlängen im Bereich von 670 bis 700 nm mit einer Beleuchtungsstärke von 20 W/m². Die Endkonzentration der Biomasse nach 24h Kultivierung betrug 3,5 g Trockenmasse/l. Der Metabolisierungsgrad der Essigsäure lag bei mehr als 95% und die Verwertung des freigesetzten CO₂ lag bei mehr als 90%.

### Beispiel 3

Das Verfahren wurde wie in Beispiel 1 durchgeführt. Die Konzentration der Suspension von *Chlorella sp.* aus dem ersten Fermenter betrug 10 g Trockenmasse/l die Konzentration der Essigsäure betrug 0,3mol/l. Die Belichtung erfolgte während der ersten 12h der Kultur mit Tageslicht mit einer Beleuchtungsstärke zwischen 5 und 20 W/m² und während der zweiten 12h der Kultur mit Kunstlicht mit Wellenlängen im Bereich von 670 bis 700 nm mit einer Beleuchtungsstärke von 20 W/m². Die Endkonzentration der Biomasse nach 24h Kultivierung betrug 30 g Trockenmasse/l. Der Metabolisierungsgrad der Essigsäure lag bei mehr als 95% und die Verwertung des freigesetzten CO₂ lag bei mehr als 95%.

### Bezugszeichenliste

- 10: Anlage
- 11: erstes Ende
- 12: zweites Ende
- 14: zellkulturgeeigneter Träger
- 16: Antriebsmittel
- 18, 22, 26, 27: Mittel zum Auftragen
- 20: mit anorganischem Carbonat gefüllte Hohlfasersuspension
- 24: Mikroorganismen- und/oder Zellkultur
- 28: schwache organische Säure
- 30: erste Lichtquelle
- 32: zweite Lichtquelle
- 34: Mittel zum Ernten
- 36, 38: Mittel zum Auffangen
- 40: Fällungsreaktor
- 42: Bioreaktor
- 44: Fermentor

## Patentansprüche

1. Verfahren zur mixotrophen Kultivierung von Mikroorganismen oder Zellen, umfassend die Schritte:
- Aufbringen einer Schicht einer Hohlfaser-Suspension auf einen Träger, wobei das Lumen der Hohlfasern mit mindestens einem anorganischen Carbonat gefüllt und/oder äußerlich beladen ist,
- Immobilisieren einer mixotrophen Mikroorganismen- oder Zellkultur auf der aufgebrachten Hohlfaserschicht, wobei sich die Mikroorganismen oder Zellen in der logarithmischen Wachstumsphase befinden,
- Zugabe einer schwachen organischen Säure mit einem pKs-Wert ≥ 3,8, deren Anion von den mixotrophen Mikroorganismen oder Zellen als Substrat verwertet wird,
- Belichten der Träger mit Tageslicht und/oder Kunstlicht,
- optional mehrfaches Wiederholen der Zugabe der schwachen organischen Säure und des Belichtens des Trägers und
- Abernten der mixotrophen Mikroorganismen oder Zellen.

2. Verfahren nach einem der vorhergehenden Ansprüche, **dadurch gekennzeichnet, dass** die Hohlfaser-Suspension mit einer Schichtdicke im Bereich von 0,1 bis 50 mm, vorzugsweise im Bereich von 1 bis 10 mm und besonders bevorzugt im Bereich von 1 bis 5 mm aufgetragen wird.

3. Verfahren nach einem der vorhergehenden Ansprüche, **dadurch gekennzeichnet, dass** die schwache organisch Säure mit einer Konzentration im Bereich von 0,001 bis 1 mol/l, vorzugsweise im Bereich von 0,01 bis 0,5 mol/l, weiter bevorzugt im Bereich von 0,01 bis 0,3 mol/l zugegeben wird.

4. Verfahren nach einem der vorhergehenden Ansprüche, **dadurch gekennzeichnet, dass** die schwache organische Säure, Essigsäure, Zitronensäure, Isozitronensäure, Oxalsäure, Milchsäure, Gluconsäure, Adipinsäure, Bernsteinsäure, Valeriansäure, Itaconsäure, cis-Aconitsäure, vorzugsweise Essigsäure ist.

5. Verfahren nach Anspruch 4, **dadurch gekennzeichnet, dass** die Essigsäure synthetisch oder durch Fermentation organischer Abfallstoffe, vorzugsweise durch Fermentation mittels eines Bakteriums der Familie der *Clostridiaceae oder Gluconobacter,* besonders bevorzugt durch Fermentation mittels *Clostridium acetobutylicum* oder *Glyconobacter oxydans* hergestellt wird.

6. Verfahren nach einem der vorhergehenden Ansprüche, **dadurch gekennzeichnet, dass** die mixotrophen Mikroorganismen und/oder Zellen phototroph sind.

7. Verfahren nach Anspruch , **dadurch gekennzeichnet, dass** als Mikroorganismen Cyanobacterien und als Zellen Mikroalgen, vorzugsweise aus der Klasse der Cryptophyceae, Chlorophyceae, Chrysophyceae, Trebouxiophyceae, Eustigmatophyceae, Bacillariophyceae, Prymnesiophyceae, besonders bevorzugt Cryptomonas sp., Chlamydomonas sp., Scenedesmus sp., Haematococcus sp., Botryococcus sp., Tetraselmis sp., Dunaliella sp., Neochloris sp., Dinobryon sp., Chlorella sp., Nannochloropsis sp., Nitzschia sp., Chaetoceros sp., Phaeodactylum sp., Pleurochrysis sp., Isochrysis sp., Arthrospira sp. oder Mischungen davon verwendet werden.

8. Verfahren nach einem der vorhergehenden Ansprüche, **dadurch gekennzeichnet, dass** das zur Belichtung verwendete Kunstlicht Wellenlängen im Bereich von 670 bis 900 nm, vorzugsweise im Bereich von 670 bis 700 nm aufweist.

9. Verfahren nach einem der vorhergehenden Ansprüche, **dadurch gekennzeichnet, dass** die Belichtung mit Tageslicht und Kunstlicht abwechselnd, vorzugsweise im Wechsel von 12h erfolgt.

10. Verfahren nach einem der vorhergehenden Ansprüche, **dadurch gekennzeichnet, dass** das Verfahren kontinuierlich durchgeführt wird, wobei als Träger ein Transportband verwendet wird.

11. Verfahren nach einem der vorhergehenden Ansprüche, **dadurch gekennzeichnet, dass** der autotrophe Anteil der Photosyntheserate im Bereich von 1/10 bis 3/5, vorzugsweise im Bereich von 1/5 bis 2/5, der Absorptionsrate der organischen Kohlenstoffquelle liegt.

12. Verfahren nach einem der vorhergehenden Ansprüche, **dadurch gekennzeichnet, dass** das anorganische Carbonat im Lumen der Hohlfasern durch Zugabe von CO₂ zu einer wässrigen Lösung an Erdalkalihydroxiden, vorzugsweise Ca(OH)₂ und/oder Mg(OH)₂ ausgefällt wird.

13. Verfahren nach Anspruch 12, **dadurch gekennzeichnet, dass** CO₂ aus dem Abgasstrom einer Biogasanlage verwendet wird.

14. Anlage (10) zur Durchführung des Verfahrens nach einem der Ansprüche 1 bis 13, aufweisend einem erstes Ende (11) und ein zweites Ende (12),
- einen zellkulturgeeigneten Träger (14) der mit einem Antriebsmittel (16) verbunden ist und das Antriebsmittel (16) eine Bewegung des Trägers (14) vom ersten Ende (11) zum zweiten Ende (12) der Anlage (10) bewirkt,
- ein Mittel (18) zum Auftragen einer mit anorganischem Carbonat gefüllten Hohlfasersuspension (20), wobei das Mittel (18) am ersten Ende (11) der Anlage (10) positioniert ist,
- ein Mittel (26) zum Auftragen einer schwachen organischen Säure (28), wobei das Mittel (26) zwischen dem Mittel (18) und dem zweiten Ende (12) der Anlage (10) positioniert ist,
- ein Mittel (22) zum Auftragen einer Mikroorganismen- und/oder Zellkultur (24), wobei das Mittel (22) zwischen dem Mittel (26) und dem zweiten Ende (12) der Anlage (10) positioniert ist,
- eine erste Lichtquelle (30), die zwischen dem Mittel (22) und dem zweiten Ende (12) der Anlage (10) positioniert ist,
- optional ein Mittel (27) zum Auftragen einer schwachen organischen Säure (28), das zwischen der ersten Lichtquelle (30) und dem zweiten Ende (12) der Anlage (10) positioniert ist,
- eine zweite Lichtquelle (32), die zwischen der ersten Lichtquelle (30) oder dem Mittel (27) und dem zweiten Ende (12) der Anlage (10) positioniert ist,
- ein Mittel (34) zum Ernten der Mikroorganismen- und/oder Zellkultur (24), das am zweiten Ende (12) der Anlage (10) positioniert ist,
- ein Mittel (36) zum Auffangen der Mikroorganismen- und/oder Zellkultur (24), das benachbart zum Mittel (34) positioniert ist und
- ein Mittel (38) zum Auffangen der Hohlfasersuspension (20), das am zweiten Ende (12) der Anlage (10) positioniert ist.

15. Anlage (10) nach Anspruch 14, **dadurch gekennzeichnet, dass** die Anlage einen Fällungsreaktor (40) zur Herstellung der mit anorganischem Carbonat gefüllten Hohlfasersuspension (20), der mit dem Mittel (18) verbunden ist, einen Bioreaktor (42) zur Herstellung der Mikroorganismen- und/oder Zellkultur (24), der mit dem Mittel (22) verbunden ist und/oder einen Fermenter (44) zur Herstellung der schwachen organischen Säure (28) aufweist, wobei der Fermenter (44) mit dem Mittel (26) und/oder dem Mittel (27) verbunden ist.
